(19) Europäisches Patentamt
European Patent Office
Office européen des brevets

(11) Numéro de publication: **0 037 319 B2**

# (12) NOUVEAU FASCICULE DE BREVET EUROPEEN

(45) Date de publication de nouveau fascicule du brevet: **28.06.95**

(51) Int. Cl.[6]: **C08B 37/10**, A61K 31/725

(21) Numéro de dépôt: **81400449.5**

(22) Date de dépôt: **20.03.81**

Le dossier contient des informations techniques présentées postérieurement au dépôt de la demande et ne figurant pas dans le présent fascicule.

## (54) Mucopolysaccharides possédant des propriétés biologiques, leur préparation et leur application en tant que médicaments.

(30) Priorité: **20.03.80 FR 8006282**

(43) Date de publication de la demande: **07.10.81 Bulletin 81/40**

(45) Mention de la délivrance du brevet: **08.04.87 Bulletin 87/15**

(45) Mention de la décision concernant l'opposition: **28.06.95 Bulletin 95/26**

(84) Etats contractants désignés: **AT BE CH DE FR GB IT LI LU NL SE**

(56) Documents cités:
**EP-A- 0 014 184**
**EP-B- 0 014 184**
**EP-B- 0 027 089**
**FR-A- 2 440 376**
**FR-A- 7 831 357**
**GB-A- 2 002 406**
**GB-A- 2 035 349**
**GB-A- 7 934 673**
**GB-A- 8 000 443**
**US-A- 3 766 167**

(73) Titulaire: **D.R.O.P.I.C. (Société Civile)**
**10, Avenue Matignon**
**F-75008 Paris (FR)**

(72) Inventeur: **Lormeau, Jean-Claude**
**5, rue Alain**
**F-76150 Maromme (FR)**
Inventeur: **Petitou, Maurice**
**27, rue du Javelor**
**F-75013 Paris (FR)**
Inventeur: **Choay, Jean**
**130, Faubourg Saint-Honoré**
**F-75008 Paris (FR)**

(74) Mandataire: **Portal, Gérard et al**
**Cabinet Beau de Loménie**
**158, rue de l'Université**
**F-75340 Paris Cédex 07 (FR)**

EP 0 037 319 B2

CHEMICAL ABSTRACTS, vol. 85, no. 13, 27 septembre 1976, page 239, réf.: 89445z Columbus, Ohio, US J.A. CIFONELLI: "Nitrous acid depolymerization of glycosaminoglycans"

BIOCHEMISTRY, 1976, vol. 15, no. 18, pages 3932-50 J.E. SHIVELY et al.: "Formation of anhydrosugars in the chemical depolymerization of heparin"

CHEMICAL ABSTRACTS, vol. 91, no. 11 10 septembre 1979, page 292, réf.: 85818z Columbus, Ohio, US U. LINDAHL et al.: "Structure of the antithrombin-binding site in heparin"

Hopwood et al., Feb. Lett.,(1976) 69, no. 1 51-54

Risenfeld et al., Fed. Proc., (1977) 36, 39-43

Lasker, Advances Exp. med. and Biol., (1976) 52, 119-130

Anderson et al., Throm. Research, (1976) 9, 575-578

Laurent et al., Biochem. J., (1978) 175, 691-701

Johnson et al., Thromb. Haemostas (1976) 35, 586-591

Whistler and Miller (ed.) Methods in Carbohydrate Chemistry Vol. VII, 139-141 (1976) (article by Cifonelli)

Lasker et al., Archives of Biochem and Biophysics (1966) 115 360-372

The Merck Index. 9th ed., (1976) 607-608

Lindahl et al., J. Biol. Chem. (1973) no. 20, 7234-7241

Javellier et al., Traité de Biochimie Générale I, (1959) 115

Shively and Conrad, Fed. Proc., (1977) 36, no. 1, 28-31

Barrowcliffe et al., Thrombosis Res. (1977) 12, 27-36

## Description

L'invention est relative à des compositions mucopolysaccharidiques possédant des propriétés biologiques leur permettant notamment de contrôler de manière hautement spécifique certaines étapes de la coagulation sanguine. Elle concerne également leur préparation et leur application en tant que principe actif de médicament.

L'invention concerne plus particulièrement des compositions de mucopolysaccharides (produits désignés ci-après par l'abréviation MPS) possédant une activité plus sélective que l'héparine, à savoir, vis-à-vis du facteur X activé, ou facteur Xa, du sang, et par là une activité antithrombotique puissante sans entraîner de risques hémorragiques pour le patient.

La recherche de voies d'accès à des produits du type évoqué ci-dessus, de mise en oeuvre aisée et à rendement élevé, a amené les inventeurs à étudier plus spécialement la dépolymérisation de l'héparine par voie chimique.

On notera que le terme héparine est utilisé dans la description et les revendications dans son sens le plus large, pour désigner indifféremment une préparation d'héparine commerciale de qualité pharmaceutique ou une héparine brute telle qu'obtenue par extraction à partir de matériaux biologiques, en particulier de tissus de mammifères.

On sait que l'héparine exerce son activité anticoagulante en potentialisant l'effet inhibiteur de l'antithrombine III (AT III), qui est une protéine du plasma, vis-à-vis des cascades de réactions enzymatiques mises en jeu au cours de la coagulation.

Etant donné que l'héparine est capable de déprimer simultanément un grand nombre de facteurs de la coagulation intervenant dans la création et le maintien des différentes formes d'hypercoagulabilité, son activité n'apparaît pas spécifique mais globale.

Si cette activité anticoagulante s'avère précieuse, elle rend toutefois délicat le rééquilibrage du système coagulation-fibrinolyse chez les patients en traitement et ce, en raison du caractère global de son action. Il s'ensuit que l'administration (dans le but de prévenir les risques d'hypercoagulation, par exemple l'apparition de thromboses postchirurgicales), de doses trop élevées de médicament anticoagulant, ou l'insuffisante sélectivité de ce médicament, peut finalement être à l'origine d'hémorragies graves.

Le document GB-A-2 035 349 décrit une fraction mucopolysaccharidique susceptible d'être obtenue à partir de l'héparine présentant les propriétés suivantes: elle est essentiellement formée de constituants dont les poids moléculaires sont compris entre 2000 et 8000 ; elle est soluble dans un milieu hydro-alcoolique (eau-éthanol) ayant un titre de 55-61° GL ; elle est insoluble dans l'alcool pur ; et elle possède des titres YW et USP dans un rapport au moins égal à 2.

Le document EP-A2-14 184 décrit la préparation de fragments hépariniques de 14 à 18 motifs, correspondant donc à des poids moléculaires compris entre 3600 et 4800 daltons, par dépolymérisation nitreuse de l'héparine en milieu organique et exige en pratique une étape supplémentaire de chromatographie d'affinité sur une colonne d'antithrombine III-sépharose.

Les inventeurs ont alors recherché des compositions de MPS comportant des chaînes de MPS dérivées de celles de l'héparine, mais plus satisfaisantes que les chaînes d'héparine au regard de leurs propriétés biologiques.

L'étude approfondie de conditions variées de dépolymérisation de l'héparine a amené les inventeurs à constater qu'il est possible,en opérant dans certaines conditions ménagées, de dépolymériser partiellement les chaînes d'héparine jusqu'à un degré correspondant à l'obtention d'une composition de MPS possédant de précieuses propriétés antithrombotiques. Ces MPS sont capables d'inhiber le facteur Xa selon un degré de sélectivité plus élevé que l'héparine alors que leur activité anticoagulante globale est plus faible que celle de l'héparine.Ils présentent en conséquence un rapport avantageux de leur titre Yin-Wessler et de leur titre USP.

On rappelle que l'activité Yin-Wessler est plus spécialement représentative de l'aptitude des produits actifs à potentialiser l'inhibition du facteur Xa du sang par l'AT III dans le test correspondant et le titre USP du pouvoir des produits actifs à inhiber la coagulation totale du sang ou du plasma.

Le titre Yin-Wessler est mesuré selon la méthode décrite par ces auteurs dans J.Lab. Clin. Med. 1976, 81, 298 à 300 et le titre USP selon la méthode décrite dans "Pharmacopea of the United States of America", pages 229 et 230 (voir également le deuxième supplément USP-NH, page 62 et le quatrième supplément USP, page 90, intitulé respectivement "Drug substances" et "Dosage forms").

L'invention a donc pour but de fournir de nouvelles compositions de MPS à activité anti-Xa élevée et présentant à l'égard du facteur Xa une spécificité remarquable dans le cadre des réactions enzymatiques successives qui caractérisent le processus de coagulation.

Elle vise également à fournir un procédé de préparation des produits du type en question à partir d'héparine, de mise en oeuvre aisée pour une exploitation industrielle et permettant l'obtention de rendements élevés.

L'invention a, en outre, pour but de fournir des principes actifs de médicaments et les médicaments eux-mêmes capables, notamment, d'inhiber le facteur Xa selon un degré de spécificité élevé alors que leur activité sur la coagulation globale peut être maintenue à un niveau très faible.

Les MPS dérivés de l'héparine selon l'invention sont susceptibles d'être obtenus à partir de l'héparine ou de fractions comportant des constituants hépariniques de poids moléculaires s'étageant, notamment, d'environ 2000 à 50 000 tels qu'obtenus par extraction à partir de tissus de mammifères.

Ces MPS sont caractérisés, notamment, par le fait qu'ils sont formés du mélange de chaînes mucopolysaccharidiques susceptible d'être obtenu par dépolymérisation nitreuse de l'héparine, ladite dépolymérisation nitreuse consistant à soumettre de l'héparine ayant des chaînes de poids moléculaires s'étageant d'environ 2000 à 50 000 à l'action de l'acide nitreux formé in situ par addition de quantités contrôlées d'un acide à un sel minéral de l'acide nitreux, cette réaction étant réalisée en milieu aqueux, à une température d'environ 0 °C à l'ambiante ; à interrompre la dépolymérisation lorsque les mucopolysaccharides présentent des poids moléculaires de 2000 à 8000 et à séparer les chaînes mucopolysaccharidiques ainsi obtenues, ledit mélange présentant les propriétés suivantes :

- il comprend une majorité de constituants ayant des poids moléculaires d'environ 2000 -8000 daltons,
- il est soluble dans un milieu hydro-alcoolique (eauéthanol) ayant un titre de 55-61 ° GL, de préférence de l'ordre de 58 ° GL,
- il tend vers l'insolubilité dans un milieu eau-éthanol ayant une teneur en alcool plus élevée,
- il est insoluble dans l'alcool pur,
- il possède des titres YW et USP respectivement dans un rapport d'au moins 3 avec une activité YW conservée par rapport à celle de l'héparine, sinon augmentée,
- il comporte une majorité de constituants avec des motifs terminaux possédant la structure de base 2,5-anhydro-D-manno dans laquelle la fonction alcool primaire en 6 est substituée ou non par un groupe $-SO_3^-$ et leurs sels pharmaceutiquement acceptables.

Le motif terminal est caractérisé par la formule générale suivante :

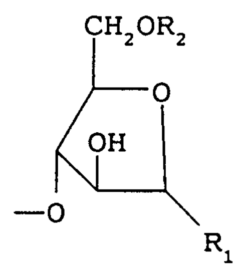

où $R_1$ représente un groupement fonctionnel choisi notamment parmi des groupements aldéhyde, alcool ou acide carboxylique ou leurs dérivés, notamment, les acétals, amides, éthers, esters ou sels correspondants et $R_2$ un atome d'hydrogène ou un groupe $SO_3^-$.

Dans un aspect avantageux de l'invention, $R_1$ est constitué par un groupe aldéhyde, acide carboxylique ou alcool.

Des MPS, selon l'invention, présentent un rapport des titres Yin-Wessler à USP d'au moins 6, avantageusement supérieur à 10.

Avantageusement, les constituants des MPS selon l'invention possèdent une activité Yin-Wessler d'environ 200-270 ui/mg et un rapport YW/USP de 10-22.

Des produits préférés selon l'invention sont caractérisés par des rapports YW/USP de l'ordre de 10 ou supérieurs, avec une activité Yin-Wessler supérieure à 250 ui/mg.

De préférence, les constituants des MPS selon l'invention possèdent une activité Yin-Wessler d'environ 250 ui/mg, un rapport YW/USP d'au moins 14 et des motifs de structure 2,5-anhydro-D-mannitol comme motifs terminaux.

De préférence encore, les MPS selon l'invention possèdent une activité Yin-Wessler d'environ 270 ui/mg, un rapport YW/USP d'environ 22 et des motifs de structure acide 2,5-anhydro-D-mannonique comme motifs terminaux.

Avantageusement, les mucopolysaccharides de l'invention se présentent sous forme de sels de calcium, de sodium ou de magnésium.

De préférence encore, les produits selon l'invention présentent un poids moléculaire d'environ 3000 à 5000 daltons.

Ces produits peuvent être obtenus par dépolymérisation de l'héparine selon le procédé défini ci-après.

Ce procédé est caractérisé en ce qu'on soumet de l'héparine ayant des chaînes de poids moléculaires s'étageant d'environ 2000 à 50 000 à l'action de l'acide nitreux formé in situ par addition de quantités contrôlées d'un acide à un sel minéral de l'acide nitreux, cette réaction étant réalisée en milieu aqueux, à une température d'environ 0 °C à l'ambiante, et lorsque le degré de dépolymérisation est atteint, qu'on interrompt la dépolymérisation lorsque les mucopolysaccharides présentent des poids moléculaires de 2000 à 8000 et qu'on sépare les mucopolysaccharides possédant une structure 2,5-anhydro-manno en bout de chaîne.

La réaction avec l'acide nitreux est effectuée dans des conditions mettant en jeu des paramètres ajustés les uns aux autres afin de permettre la réalisation d'une dépolymérisation partielle du matériau héparinique de départ jusqu'à l'obtention d'un mélange formé en majeure partie de produits ayant des titres Yin-Wessler et USP dans un rapport d'au moins 3, avantageusement supérieur à 6, voire à 10. Les groupes réducteurs terminaux peuvent repondre à la formule:

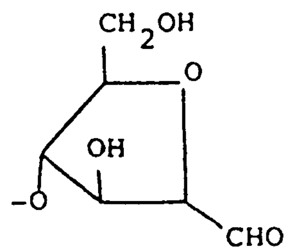

Ces groupes terminaux résultent de l'action de l'acide nitreux au niveau des motifs N-sulfate-glucosamine de l'héparine.

La dépolymérisation de l'héparine peut être égalememt avantageusement conduite de manière à obtenir une composition formée d'espèces moléculaires dont la majeure partie, avantageusement plus de 80% en poids, par rapport à l'héparine initiale, est soluble dans un milieu hydro-alcoolique (eau-éthanol) ayant un titre de 55-62° GL, de préférence de l'ordre de 58° GL. Les conditions de dépolymérisation peuvent être également ajustées par rapport au poids moléculaire de la majeure partie des espèces présentes dans le mélange de dépolymérisation. Ces conditions seront alors fixées afin d'obtenir une majorité d'espèces de poids moléculaire d'environ 2000 à 8000 daltons (notamment de 3000 à 5000 daltons).

Lorsque le degré de dépolymérisation souhaité est atteint, on sépare du mélange de dépolymérisation les MPS qui sont précipitables par un solvant alcoolique et on les récupère.

D'une manière avantageuse, il s'agit de MPS qui constituent en quelque sorte une héparine "améliorée". Ils possèdent, en effet, de précieuses propriétés biologiques, en particulier un titre USP plus faible que celui de l'héparine de départ alors que l'activité Yin-Wessler initiale présente dans l'héparine est conservée, sinon augmentée.

En outre, d'une manière avantageuse, le procédé de l'invention peut être mis en oeuvre avec une héparine de qualité pharmaceutique, telle que disponible dans le commerce, cette héparine étant formée d'une majorité d'espèces qui sont insolubles dans un milieu hydro-alcoolique ayant un titre de 55-61 °GL, de préférence de 58 °GL.

En effectuant la dépolymérisation partielle comme indiqué ci-dessus, on recouvre des chaînes hépariniques possédant des propriétés biologiques plus sélectives. D'une manière générale cette récupération de chaînes actives s'effectue avec des rendements élevés, jusqu'à 80% ou plus par rapport à la quantité d'héparine traitée.

Ce mélange de chaînes actives peut être utilisé tel quel, après avoir subi, compte-tenu des applications thérapeutiques envisagées, des opérations de purification classiques telles que la dialyse, la mise en contact avec des résines échangeuses d'ions, en particulier des opérations de chromatographie.

Selon une disposition supplémentaire, avantageusement mise en oeuvre afin de disposer d'un mélange de MPS possédant des groupements terminaux de plus grande stabilité, on soumet le mélange précédemment obtenu à un traitement permettant la transformation du groupe aldéhyde en un groupement fonctionnel plus stable, notamment en un groupement acide ou alcool.

Dans un mode préféré de réalisation du procédé de l'invention, on met avantageusement en oeuvre comme matière première une héparine possédant un poids moléculaire de 2000 à 50 000 environ.

Il peut s'agir d'une héparine de qualité pharmaceutique classique, injectable, ou d'une héparine brute telle qu'elle est obtenue a l'issue des opérations d'extraction de ceprincipe actif à partir de tissus ou d'organes de mammifères, notamment de mucus d'intestins ou de poumons, par exemple, de porc ou de boeuf. Elle peut encore être constituée par les produits qui sont normalement écartés lors de la purification d'une héparine en vue de l'obtention d'une héparine de qualité injectable et d'activité spécifique plus élevée.

L'héparine mise en oeuvre est soumise à l'action d'un agent chimique capable, dans les conditions ménagées utilisées,de dépolymériser partiellement l'héparine en conduisant à des chaînes biologiquement actives telles qu'évoquées ci-dessus.

On a plus spécialement recours à l'acide nitreux $HNO_2$. Cet acide agit au niveau des motifs N-sulfate-glucosamine de l'héparine et les transforme en groupements de structure 2,5-anhydro-D-mannose. Avantageusement, on produit l'acide nitreux in situ par addition, selon des quantités contrôlées, d'un acide à un dérivé de l'acide nitreux, en particulier à un sel bu un éther-sel.Dans un mode de réalisation avantageux de l'invention,on utilise un sel alcalin ou alcalino-terreux, plus particulièrement le nitrite de sodium $NaNO_2$. Pour engendrer in situ l'acide nitreux, on ajoute des quantités contrôlées d'un acide comportant, de préférence, des anions physiologiquement compatibles, tel que l'acide acétique ou encore l'acide chlorhydrique.

L'action de l'acide nitreux sur l'héparine est avantageusement effectuée en milieu aqueux.

La mise en oeuvre de ces dispositions présente un intérêt tout particulier au regard des applications biologiques envisagées.

Le milieu aqueux dans lequel onréalise la dépolymérisation constitue, en effet, un milieu physiologiquement acceptable, ce qui permet d'éviter tout problème concernant l'élimination d'un solvant nuisible pour lesdites applications.En outre, le sel qui se forme dans le milieu lors de la production d'acide nitreux est un sel hydrosoluble, à savoir, du chlorure de sodium, dont la présence n'apparaît pas non plus gênante. Les étapes de purification ultérieure du mélange de dépolymérisation évoquées ci-dessus s'en trouvent donc facilitées, aucun composé ou espèce organique n'étant introduit dans le milieu réactionnel.

Les divers paramètres qui interviennent lors d'une réaction chimique, en particulier les concentrations en réactifs, la durée, la température et le pH, sont ajustés les uns aux autres afin d'obtenir les produits désirés dans des conditions expérimentales les plus satisfaisantes.

On sait, à cet égard, combien ces différents paramètres sont généralement étroitement liés.

Il est clair que la modification d'un de ces facteurs peut entraîner un ajustement d'un ou de plusieurs autres facteurs en conséquence.

L'étude de ces conditions expérimentales par la demanderesse a montré qu'il est avantageux de mettre en oeuvre les réactifs selon des quantités conduisant à une concentration finale en héparine de l'ordre de 1 à 10 g de préférence, de 1,5 à 5 g, notamment voisine de 2 g pour 100 ml de milieu réactionnel, la concentration finale en nitrite de sodium pouvant varier de 0,02 M à 0,1 M environ et étant de préférence de l'ordre de 0,05 M. L'acide chlorhydrique est utilisé en quantité suffisante pour l'obtention d'un pH du milieu réactionnel de l'ordre de 2 à 3, avantageusement de 2,2 à 2,7 de préférence de 2,5.

En opérant à une température de l'ordre de 0 à 10 °C, de préférence de l'ordre de 4 °C, on laisse réagir pendant une durée suffisante pour l'obtention du degré de dépolymérisation souhaité. A titre indicatif, une incubation de 10 mn environ est apparue suffisante quand on opère à 4 °C.

On interrompt alors l'opération de dépolymérisation.

A cet effet, on a recours avantageusement à une augmentation du pH du milieu.

L'addition d'un agent alcalin, par exemple, de soude, en quantité suffisante pour obtenir un pH au moins neutre ou légèrement alcalin, permet l'interruption souhaitée de la réaction de dépolymérisation.

On sépare ensuite les mucopolysaccharides qui précipitent avec un solvant alcoolique.

L'utilisation d'éthanol absolu, à raison d'environ 5 volumes, permet d'obtenir la séparation souhaitée.

Le précipité est récupéré et, en vue de son utilisation, lavé et séché.

Par mise en oeuvre des dispositions indiquées ci-dessus, on isole des MPS possédant des titres YW/USP dans un rapport d'au moins 3/1, avec un titre en activité Yin-Wessler supérieur à 200 ui/mg, et ce, à partir d'héparine de départ ayant des rapports YW/USP de l'ordre de 1.

Selon une disposition supplémentaire, on transforme les fonctions aldéhyde des groupes terminaux réducteurs en groupements fonctionnels plus stables tels que des groupements alcool ou acide , ce qui conduit à des chaînes de MPS terminées pour la plupart par des motifs de structure 2,5-anhydro-D-mannitol ou acide 2,5-anhydro-D-mannonique.

Pour transformer les groupes terminaux 2,5-anhydro-D-mannose, en groupes 2,5 anhydro-D-mannitol, on soumet les produits précédemment recueillis du précipité à l'action d'un agent réducteur en mettant en oeuvre les conditions permettant d'obtenir au moins en partie la transformation souhaitée.

L'agent réducteur est choisi parmi ceux utilisés habituellement pour la transformation des groupes aldéhyde en groupes alcool. Parmi ces agents, il apparaît avantageux de mettre en oeuvre un borohydrure métallique.

On réalise avantageusement la réaction en milieu aqueux en présence de borohydrure de sodium ou de potassium durant plusieurs heures.

En opérant à température ambiante, de préférence sous agitation, il apparaît suffisant de laisser le mélange réagir durant environ 4 heures. On observe une augmentation du pH du milieu réactionnel. Ce pH peut atteindre une valeur de l'ordre de 10 dans le cas de l'utilisation de Na $BH_4$ où l'on observe une libération de soude.

Afin de détruire le borohydrure qui n'a pas réagi, on abaisse le pH par addition d'acide.

Dans le cas particulier considéré, il apparaît avantageux d'abaisser le pH à 4 par addition, par exemple, d'acide acétique.

On rèajuste ensuite le pH à une valeur voisine de la neutralité, en particulier de l'ordre de 7,5 par addition d'un agent alcalin, par exemple, de soude.

On récupère du milieu réactionnel ceux des produits qui peuvent être précipités par de l'alcool et l'on récupère le précipité formé qui renferme les produits recherchés,dans lesquels la terminaison de structure 2,5-anhydro-D-mannose a été transformée en structure 2,5-anhydro-D-mannitol.

La précipitation en question peut être effectuée par addition d'alcool éthylique absolu, avantageusement, à raison de 5 volumes.

Pour isoler les produits réduits recherchés, on procède avantageusement à une centrifugation et l'on recueille le culot de centrifugation qui est ensuite, si on le désire, lavé et séché.

L'étude des MPS ainsi obtenus montre qu'ils possèdent des titres YW/USP dans un rapport de l'ordre de 10 ou supérieur, avec des titres en activité Yin-Wessler supérieurs à 200 ui/mg, avantageusement supérieurs à 250 ui/mg.

Selon une variante, on transforme les groupes 2,5-anhydro-D-mannose en groupes acide 2,5-anhydro-D-mannonique.

On fait réagir dans les conditions nécessaires pour obtenir la transformation souhaitée les produits comportant les groupes terminaux 2,5-anhydro-D-mannose avec un agent oxydant choisi parmi ceux habituellement utilisés pour la transformation des groupes aldéhyde en groupes acide carboxylique, en particulier les permanganates.

La réaction est avantageusement réalisée en milieu aqueux à un pH supérieur à la neutralité.

L'oxydation des groupements aldéhyde en groupements acide entraîne une chute du pH qu'il est avantageux d'ajuster constamment au cours de la réaction.

En opérant à la température ambiante, on obtient au bout de 15 heures environ l'oxydation souhaitée. On récupère du mélange réactionnel les produits qui précipitent avec un solvant alcoolique.

Le précipité est ensuite avantageusement lavé et séché.

Il est entendu que les indications de poids moléculaires qui précèdent (et qui suivent, notamment dans les exemples) découlent des mesures de temps de rétention de solutions ayant une teneur déterminée de matière étudiée, dans des expériences de gel-perméation à travers une colonne de gel, dans des conditions d'élution également déterminées, les logarithmes de ces indications de poids moléculaire étant dans la même relation de proportionnalité vis-à-vis des temps de rétention mesurés susdits, que le sont ceux des poids moléculaires de 4000, 6500, 16000 et 31 000 respectivement, de polystyrène-sulfonates de sodium étalons, notamment ceux commercialisés par la société dite CHROMPACK (Orsay-les-Ulis, France), vis-à-vis de leur temps de rétention respectifs, mesurés dans un système et sous des conditions de gel-perméation identiques.

Dans la mesure où les produits traités quels que soit le degré de purification atteint, se trouvent à l'état de sels d'un métal physiologiquement acceptable, tel que le sodium, ils peuvent ensuite être transformés en des sels mixtes ou simples contenant un autre métal physiologiquement acceptable, tel que le calcium, par tout procédé applicable aux sels d'héparine. Avantageusement, on pourra avoir recours au procédé décrit dans le brevet français No 73 13580 déposé le 13 avril 1973 au nom de la demanderesse. On rappelle que ce procédé consiste essentiellement, partant par exemple d'un sel de sodium d'héparine, à mettre celui-ci en contact avec un sel différent d'un autre métal physiologiquement acceptable, par exemple, le chlorure de calcium, au sein d'une solution, à procéder ensuite à la séparation des ions métalliques non liés à l'héparine (par exemple, par précipitation alcoolique ou dialyse) et, dans la mesure où le taux de substitution atteint n'est pas suffisant, à remettre en contact, au sein d'une solution, le sel

mixte d'héparine obtenu au terme du premier contact, avec une nouvelle dose de l'autre sel, notamment du chlorure de calcium, selon le taux de substitution final désiré.

D'autres caractéristiques et avantages de l'invention apparaîtront dans la description qui suit :

EXEMPLE 1 - Dépolymérisation partielle de l'héparine et obtention d'un mélange de MPS possédant des activités Yin-Wessler d'au moins 240 ui/mg,un rapport de leur titre YW à leur titre USP d'au moins 14 et des groupes réducteurs terminaux ayant la structure du 2,5-anhydro-D-mannose, à savoir :

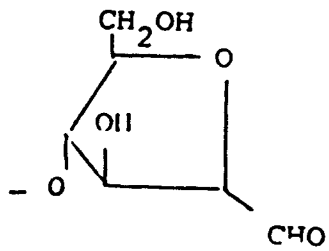

le groupe hydroxyle primaire en 6 pouvant être diversement substitué notamment par un groupe $SO_3^-$ .

On soumet avantageusement une matière première héparinique à l'action ménagée de $HNO_2$ en procédant comme suit.

On dissout 60 g d'héparine commerciale ayant un rapport YW/USP voisin de 1 et un titre USP de 160 ui/mg dans 3 l d'eau distillée à + 4°C.

On ajoute du nitrite de sodium $NaNO_2$ en quantité suffisante pour avoir une solution 0,05M, soit 10,35 g puis, on ajuste le pH à 2,5 à l'aide d'acide chlorhydrique pur et on agite à +4°C pendant 10 mn. On ajuste alors le pH à 7,5 à l'aide de soude 5N.

Par addition de 5 volumes d'éthanol pur (soit 15 500 ml), on précipite les produits de la réaction. On récupère par centrifugation le précipité formé. On le lave avec de l'éthanol et on le sèche à 60°C sous vide profond. On recueille 60 g de produit présentant les caractéristiques suivantes :

| | |
|---|---|
| titre USP | 17 ul/mg |
| titre YW | 240 ul/mg |
| titre AP TT * | 12 ul/mg |

* abréviation des termes anglais "activated partial thrombosplastin time"(équivalent du Temps de Céphaline Kaolin - Caen J. et al. L'hémostase, édit. Expansion Scientifique (1976-169-170)

Dans un autre essai, on procéde comme indiqué ci-dessus mais on utilise comme matière de départ un autre lot d'héparine titrant 165 ui/mg en unités USP et présentant un rapport YW/USP de l'ordre de 1.

On en dissout 3 g dans 150 ml d'eau distillée, à +4°C, 517 mg de Na $NO_2$ étant ensuite ajoutés au milieu réactionnel.

A l'issue du traitement réalisé comme précédemment décrit, on récupère 2,8 g de produit ayant un titre USP de 24 ui/mg et un titre en activité Yin Wessler de 250 ui/mg.

Selon encore un autre essai, réalisé dans les conditions définies plus haut, on met en oeuvre 50 g d'héparine de titre USP, 158 ui/mg avec un rapport YW/USP voisin de 1.

On les dissout dans 2500 ml d'eau distiliée et à +4°C on ajoute 8,625 g de $NaNO_2$. A l'issue du traitement on récupère 46 g de produit possédant les caractéristiques suivantes :

| | |
|---|---|
| titre USP | 13 ui/mg |
| titre en activité Y & W | 270 ui/mg |
| titre APTT | 7 ui/mg |

EXEMPLE II - Dépolymérisation partielle de l'héparine et obtention d'un mélange de MPS possédant des activités Yin-Wessler de 250 ui/mg un rapport de leur titre YW à USP de 15 et des groupes terminaux ayant la structure du 2,5 anhydro-D-mannitol, à savoir :

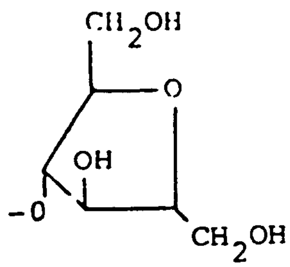

l'alcool primaire en 6 pouvant être substitué comme évoqué ci-dessus.

On dissout 47 g du produit obtenu dans l'exemple I dans 1200 ml d'eau distillée à température ambiante. Sous forte agitation, on ajoute 7 g de borohydrure de potassium $KBH_4$. Cette agitation est maintenue 2 heures à température ambiante puis on ajoute de l'acide acétique au milieu réactionnel afin d'abaisser le pH à 4,0 et de détruire ainsi le $KBH_4$ qui n'a pas été consommé.

On soumet le milieu à agitation pendant 30 mn, puis on ajuste le pH à 7,5 avec de la soude 5N.

On ajoute 5 volumes d'alcool au milieu réactionnel.

Le précipité formé est recueilli, essoré, lavé avec de l'éthanol pur et séché sous vide à 60 °C.

On récupère 46,5 g de produit présentant les caractéristiques suivantes :

| | |
|---|---|
| titre USP | 17 ui/mg |
| titre en activité Y & W | 250 ui/mg |
| titre APTT | 11 ui/mg |

EXEMPLE III - Dépolymérisation partielle de l'héparine et obtention d'un mélange de MPS possédant des activités Yin-Wessler de 270 ui/mg, un rapport de leur titre YW à leur titre USP de 22 et des groupes terminaux ayant la structure du 2,5 anhydro-D-mannitol, à savoir

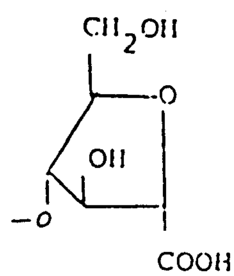

le groupement hydroxyie primaire en 6 pouvant être substitué comme déjà indique.

On dissout 40 g du produit obtenu selon le dernier essai rapporté dans l'èxemple I dans 400 ml d'eau distillée, à température ambiante.

On ajuste le pH à 8,5 avec de la soude 5N puis on ajoute 2 g de permanganate de potassium $KMnO_4$ dissous dans 40 ml d'eau.

On soumet le mélange réactionnel à une forte agitation pendant 15 heures. Durant cette agitation, on ajuste constamment le pH du mélange à 8,5 avec de la soude 5N.

Au bout de 15 heures, on ajoute 0,2 volume d'alcool (90 ml) pour achever de réduire le $KMnO_4$ qui n'a pas réagi.

On laisse le mélange réactionnel au repos durant 1 heure. On élimine par centrifugation le précipité de $MnO_2$ formé. Par précipitation avec de l'éthanol, on récupère les produits de la réaction, on les lave et on les sèche. On recueille ainsi 35 g de produit possédant les caractéristiques suivantes :

| | |
|---|---|
| titre USP | = 12 ui/mg |
| titre en activité Y & W | = 270 ui/mg |
| titre APTT | = 8 ui/mg. |

Les produits obtenus selon l'invention peuvent être utilisés sous toutes les formes de sels thérapeutiquement utiles, notamment de métaux physiologiquement acceptables (sels de sodium, calcium, magnésium ou sels mixtes). On passe éventuellement de l'un à l'autre par le procédé décrit dans la demande de brevet FR 73 13 580 déposée le 13 avril 1973, au nom de la demanderesse.

L'étude pharmacologique des produits de l'invention a montré qu'ils possèdent une action nettement plus sélective, notamment au niveau de l'inhibition du facteur Xa, que celle de l'héparine.

De plus, ils présentent l'avantage de ne pas provoquer d'agrégation plaquettaire avec le sang de patients sur lesquels l'héparine provoque nne telle réaction.

A titre indicatif, on rapporte ci-après les résultats obtenus en testant les produits de l'invention dans différents systèmes.

1) On a étudié in vitro, sur sang humain, leur activité vis-à-vis des plaquettes. On a ainsi constaté qu'à des doses équivalentes à celles de l'héparine, ces produits avaient l'avantage de ne pas provoquer d'agrégation plaquettaire du sang de patients pour lesquels l'héparine provoquait une telle réaction.

2) Le temps de saignement chez des animaux traités par des MPS conformes à l'invention a été compare à celui obtenu avec de l'héparine.

A cet effet, on a effectué une blessure sur l'abdomen d'un rat préalablement anesthésié et rasé. La coupure a été effectuée par arrachage d'un lambeau de peau que l'on a recouvert d'une gaze pendant 10 mn. Après extraction du sang avec de l'eau distillée, on a mesuré la quantité d'hémoglobine par une méthode spectrophotomètrique. On a exprimé les résultats en pourcentage par rapport à la quantité d'hémoglobine extraite pour un animal ayant reçu un placébo.

En administrant 5 à 10 mg/kg des produits selon l'invention, la quantité d'hémoglobine recueillie a été de 130% de celle du placébo, alros que pour des doses d'héparine de 1,2 et 4 mg/kg, on a obtenu des pourcentages de 170, 180 et 325% respectivement.

3) On a enfin étudié l'activité antithrombotique in vivo chez le lapin,selon le modèle de Wessler : on a administré au lapin 20 u/kg de complexe de prothrombine concentrée (Konyne de Laboratoire Cutter), puis 0,5 ml de venin de vipère Russel pour provoquer la formation de thrombine. L'effet des produits selon l'invention a été étudié, comparativement à l'héparine lorsqu'on les administre 15 mn avant l'injection des produits thrombogènes. Les résultats obtenus sont les suivants :

| DOSE | HEPARINE | PRODUITS DE L'INVENTION |
|---|---|---|
| 500 u.Y & W/kg | protection totale | protection totale |
| 250 u.Y & W/kg | protection totale | protection totale |
| 125 u.Y & W/kg | protection partielle | protection partielle |
| 62,5 u.YW/kg | pas de protection | faible protection |

On constate donc au vu des résultats de ces essais que les produits de l'invention possèdent une activité antithrombotique au moins équivalente à celle de l'héparine et avantageusement exercent une réaction moindre vis-à-vis des plaquettes et permettent de réduire le temps de saignement. Ces résultats mettent donc en évidence que les produits de l'invention exercent une action très faible sur l'activité anticoagulante globale mais sont doués d'une activité antithrombotique de grand intérêt.

Avantageusement, les produits de l'invention sont dépourvus de toxicité.

L'administration de 10 000 u/kg (titre Yin-Wessler), par exemple, des produits conformes à l'exemple 1 ne provoque chez le lapin aucune réaction toxique, ni d'effet pyrogénique dans letest de pyrogénicité chez le lapin conforme à la pharmacopée française.

L'invention est donc relative à des préparations pharmaceutiques qui renferment lesdites compositions de MPS ayant notamment une activité d'au moins 200 ui/mg, et un rapport Yw/USP supérieur à 6.

Elle est plus particulièrement relative à des préparations pharmaceutiques dépourvues de substances pyrogéniques lorsque nécessaire et contenant une quantité efficace de principes actifs en association avec des excipients pharmaceutiques.

En particulier, elle concerne les compositions dans lesquelles le véhicule pharmaceutique est approprié pour l'administration par voie orale. Des formes d'administration de l'invention appropriées pour l'administration par voie orale peuvent être avantageusement des gélules gastrorésistantes, des comprimés ou tablettes ou des pilules.

D'autres compositions pharmaceutiques comprennent ces compositions de MPS en association avec les excipients appropriés pour l'administration par voie rectale. Des formes d'administration correspondantes sont constituées par des suppositoires.

D'autres formes d'administration sont constituées par des aérosols ou des pommades.

L'invention concerne également des compositions pharmaceutiques injectables stériles ou stérilisables.

Ces solutions renferment avantageusement 1000 à 100 000 u (Yin-Wessler)/ml de produits de l'invention, de préférence de 5000 à 50 000, par exemple de 25 000 u/ml, lorsque ces solutions sont destinées à l'injection par voie sous-cutanée. Elles peuvent contenir, par exemple, de 500 à 10 000, notamment 5000 u/ml de compositions de MPS lorsqu'elles sont destinées à l'injection par voie intraveineuse ou par perfusion.

Avantageusement, de telles préparations pharmaceutiques sont présentées sous la forme de seringue non récupérables, prêtes à l'emploi.

L'invention concerne également les compositions pharmaceutiques contenant lesdites compositions de MPS en association avec u n autre principe actif, utilisable en particulier pour la prophylaxie et le traitement de thrombose, tel qu'un agent veinotonique comme la dihydroergotamine, un sel d'acide nicotinique ou un agent thrombolytique comme l'urokinase.

Les compositions pharmaceutiques de l'invention sont particulièrement adaptées pour le contrôle (préventif ou curatif) de certaines étapes de la coagulation du sang chez l'homme ou l'animal, notamment dans le cas où le patient est soumis à des risques d'hypercoagulabilité résultant notamment de la libération par l'organisme de thromboplastines, par exemple, de thromboplastines tissulaires (opérations chirurgicales, processus atheromateux, développement de tumeurs et troubles de la coagulation par des activateurs bactériens ou enzymatiques, etc...).

Afin d'illustrer l'invention, on indique, ci-après un exemple de posologie utilisable chez l'homme : cette posologie comprend, par exemple, l'administration au patient de 1000 à 25000 u par voie sous-cutanée, deux ou trois fois par jour, selon le niveau des risques d'hypercoagulabilité ou la condition thrombotique du patient, ou de 1000 à 25000 u/24 heures par voie intraveineuse, en administrations discontinues à intervalles réguliers, ou continues par perfusion, ou encore de 1000 à 25000 u (trois fois par semaine) par voie intramusculaire (ces titres sont exprimés en unités Yin-Wessler). Ces doses peuvent être standardisées ou ajustées pour chaque patient en fonction des résultats et des analyses de sang effectuées auparavant, la nature des affections dont il souffre et, d'une manière générale, son é tat de santé.

L'invention se rapporte également à l'application des compositions définies ci-dessus, à la constitution de réactifs biologiques, utilisables en laboratoires notamment comme éléments de comparaison pour l'étude d'autres substances dont on souhaite tester l'activité anticoagulante, notamment au niveau de l'inhibition du facteur Xa.

## Revendications

**1.** Mucopolysaccharides dérivés de l'héparine, caractérisés par le fait qu'ils sont formés du mélange de chaînes mucopolysaccharidiques susceptible d'être obtenu par dépolymérisation nitreuse de l'héparine, ladite dépolymérisation nitreuse consistant à soumettre de l'héparine ayant des chaînes de poids moléculaires s'étageant d'environ 2000 à 50 000 à l'action de l'acide nitreux formé in situ par addition de quantités contrôlées d'un acide à un sel minéral de l'acide nitreux, cette réaction étant réalisée en milieu aqueux, à une température d'environ 0 °C à l'ambiante, à interrompre la dépolymérisation lorsque les mucopolysaccharides présentent des poids moléculaires de 2000 à 8000 et à séparer les chaînes mucopolysaccharidiques ainsi obtenues, ledit mélange présentant les propriétés suivantes :

- il comprend une majorité de constituants ayant des poids moléculaires d'environ 2000 -8000 daltons,
- il est soluble dans un milieu hydro-alcoolique (eau-éthanol) ayant un titre de 55-61 ° GL, de préférence de l'ordre de 58 ° GL,
- il tend vers l'insolubilité dans un milieu eau-éthanol ayant une teneur en alcool plus élevée,
- il est insoluble dans l'alcool pur,
- il possède des titres YW et USP respectivement dans un rapport d'au moins 3 avec une activité YM conservée par rapport à celle de l'héparine, sinon augmentée,
- il comporte une majorité de constituants avec des motifs terminaux possédant la structure de base 2,5-anhydro-D-manno dans laquelle la fonction alcool primaire en 6 est substituée ou non par un groupe $-SO_3^-$ et leurs sels pharmaceutiquement acceptables.

**2.** Mucopolysaccharides selon la revendication 1, caractérisés en ce qu'ils présentent un rapport des titres Yin-Wessler à USP d'au moins 6, avantageusement supérieur à 10.

**3.** Mucopolysaccharides selon la revendication 1 ou 2, caractérisés par le fait que le motif terminal répond à la formule :

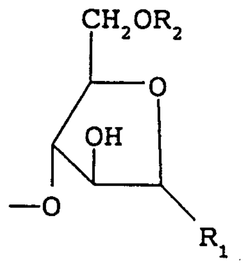

dans laquelle $R_1$ représente un groupement fonctionnel choisi notamment parmi des groupements aldéhyde, alcool ou acide carboxylique ou leurs dérivés, notamment les acétals, amides, éthers, esters ou sels correspondants, et $R_2$ représente un atome d'hydrogène ou un groupe $-SO_3^-$ et les sels physiologiquement acceptables.

**4.** Mucopolysaccharides selon la revendication 3, caractérisés en ce que $R_1$ représente un groupe $-CH_2OH$.

**5.** Mucopolysaccharides selon la revendication 2, caractérisés en ce que lesdits constituants possèdent une activité Yin-Wessler d'environ 200-270 ui/mg et un rapport YW/USP de 10-22.

**6.** Mucopolysaccharides selon la revendication 2, caractérisés en ce que lesdits constituants possèdent une activité Yin-Wessler d'environ 250 ui/mg, un rapport YM/USP d'au moins 14 et des motifs de structure 2,5-anhydro-D-mannitol comme motifs terminaux.

**7.** Mucopolysaccharides selon la revendication 2, caractérisés en ce que lesdits constituants possèdent une activité Yin-Wessler d'environ 270 ui/mg, un rapport YW/USP d'environ 22 et des motifs de structure acide 2,5-anhydro-D-mannonique comme motifs terminaux.

**8.** Mucopolysaccharides selon l'une quelconque des revendications 1 à 7, caractérisés en ce qu'ils se présentent sous forme de sels de calcium, de sodium ou de magnésium.

**9.** Mucopolysaccharides selon l'une des revendications 1 à 8, caractérisés en ce qu'ils présentent un poids moléculaire d'environ 3000 à 5000 daltons.

**10.** Procédé pour l'obtention de mucopolysaccharides dérivés de l'héparine formés d'un mélange de chaînes mucopolysaccharidiques présentant les propriétés suivantes :

- il comprend une majorité de constituants ayant des poids moléculaires d'environ 2000 - 8000 daltons,
- il est soluble dans un milieu hydro-alcoolique (eau-éthanol) ayant un titre de 55-61° GL, de préférence de l'ordre de 58° GL,
- il tend vers l'insolubilité dans un milieu eau-éthanol ayant une teneur en alcool plus élevée,
- il est insoluble dans l'alcool pur,
- il possède des titres YW et USP respectivement dans un rapport d'au moins 3 avec une activité YW conservée par rapport à celle de l'héparine, sinon augmentée,
- il comporte une majorité de constituants avec des motifs terminaux possédant la structure de base 2,5-anhydro-D-manno dans laquelle la fonction alcool primaire en 6 est substituée ou non par un groupe $-SO_3^-$ et de leurs sels pharmaceutiquement acceptables,

caractérisé par le fait qu'on soumet de l'héparine ayant des chaînes de poids moléculaires s'étageant d'environ 2000 à 50 000 à l'action de l'acide nitreux formé in situ par addition de quantités contrôlées d'un acide à un sel minéral de l'acide nitreux, cette réaction étant réalisée en milieu aqueux, à une température d'environ 0°C à l'ambiante, et lorsque le degré de dépolymérisation est atteint, qu'on interrompt la dépolymérisation lorsque les mucopolysaccharides présentent des poids moléculaires de 2000 à 8000 et qu'on sépare les mucopolysaccharides possédant une structure 2,5-anhydro-manno en bout de chaîne.

**11.** Procédé selon la revendication 10, caractérisé par le fait que le sel minéral de l'acide nitreux mis en oeuvre est un sel alcalin ou alcalinoterreux.

**12.** Procédé selon la revendication 11, caractérisé en ce que le sel minéral de l'acide nitreux est du nitrite de sodium.

**13.** Procédé selon la revendication 12, caractérisé par le fait que l'acide nitreux est formé in situ par addition au nitrite de sodium d'un acide possédant des anions physiologiquement acceptables, tels que l'acide acétique et de préférence l'acide chlorhydrique.

**14.** Procédé selon l'une quelconque des revendications 11 à 13, caractérisé par le fait que la concentration finale en héparine est avantageusement de 1 à 10 g, de préférence de 1,5 à 5 g, et notamment voisine de 2 g pour 100 ml de milieu réactionnel, la concentration finale en nitrite de sodium pouvant varier de 0,02M à 0,1M, et étant de préférence de 0,05M environ.

**15.** Procédé selon la revendication 13 ou 14, caractérisé par le fait que, pour former in situ l'acide nitreux, on ajoute de l'acide chlorhydrique, en quantité contrôlée et suffisante pour l'obtention d'un pH de l'ordre de 2 à 3, avantageusement de 2,2 à 2,7 , de préférence de 2,5.

**16.** Procédé selon l'une quelconque des revendications 10 à 15, caractérisé par le fait qu'on opère à une température de l'ordre de 0 à 10 °C, de préférence de 4 °C, pendant une durée suffisante pour l'obtention du degré de dépolymérisation désiré, cette durée étant d'environ 10 mn lorsqu'on opère à 4 °C.

**17.** Procédé selon l'une quelconque des revendications 10 à 16, caractérisé par l'étape supplémentaire selon laquelle on soumet le mélange de mucopolysaccharides à groupes terminaux de structure 2,5-anhydro-D-mannose à une opération de réduction à l'aide d'un agent réducteur choisi parmi ceux utilisés habituellement pour la transformation des groupes aldéhyde en groupes alcool, en particulier avec un borohydrure métallique, et on récupère les produits réduits obtenus dont les groupes terminaux possèdent la structure 2,5-anhydro-D-mannitol.

**18.** Procédé selon l'une quelconque des revendications 10 à 16, caractérisé par l'étape supplémentaire selon laquelle on soumet le mélange de mucopolysaccharides à groupes terminaux de structure 2,5-anhydro-D-mannose à l'action d'un agent d'oxydation choisi parmi ceux utilisés habituellement pour la transformation des groupes aldéhyde en groupes acide et on récupère les produits oxydés obtenus dont les groupes terminaux possèdent la structure acide 2,5-anhydro-D-mannonique.

**19.** Médicaments caractérisés par le fait qu'ils comprennent une quantité efficace d'au moins un mucopolysaccharide formé d'un mélange de chaînes mucopolysaccharidiques selon l'une quelconque des revendications 1 à 9, en association avec un véhicule pharmaceutique.

**20.** Médicaments selon la revendication 19, caractérisés en ce qu'ils se présentent sous des formes appropriées pour l'administration par voie orale, avantageusement sous forme de gélules gastrorésistantes, de comprimés ou tablettes, de pilules, ou encore sous forme appropriée pour l'administration par voie rectale, sous forme de suppositoires, ou bien sous forme d'aérosols ou de pommades, ou encore sous forme de compositions injectables.

**21.** Médicaments selon la revendication 20, caractérisés en ce qu'ils se présentent sous forme de compositions pharmaceutiques injectables renfermant de 1000 à 100 000 ui (Yin-Wessler) par ml de produits, de préférence de 5000 à 50 000, notamment de 25000 ui/ml lorsque ces solutions sont destinées à l'injection par voie sous-cutanée et de 500 à 10 000, notamment de 5000 ui/ml de produits lorsqu'elles sont destinées à l'injection par voie intraveineuse ou par perfusion.

**Claims**

**1.** Mucopolysaccharides derived from heparin, characterized in that they are formed from the mixture of mucopolysaccharide chains obtainable by nitrous depolymerization of heparin, said nitrous depolymerization consisting in subjecting heparin having chains of molecular weights ranging from about

2000 to 50000, to the action of the nitrous acid formed in situ by the addition of controlled quantities of an acid to an inorganic salt of the nitrous acid, this reaction being made in an aqueous medium, at a temperature of from about 0 °C to ambient, in interrupting depolymerization when the mucopolysaccharides present molecular weights of 2000 to 8000, and in separating the mucopolysaccharide chains thus obtained, said mixture presenting the following properties:

- it comprises a majority of components having molecular weights of about 2000 - 8000 daltons,
- it is soluble in a water and alcohol medium (water and ethanol) having a titer of 55-61 ° GL, preferably of the order of 58 ° GL,
- it tends towards insolubility in a water and ethanol medium having a higher alcohol content,
- it is insoluble in pure alcohol,
- it has YW and USP titers respectively in a ratio of at least 3 with a YW activity maintained, if not increased, with respect to that of heparin,
- it comprises a majority of components having terminal units having the basic 2,5-anhydro-D-manno structure in which the primary alcohol function at 6 may or may not be substituted by an $-SO_3^-$ group, and their pharmaceutically acceptable salts.

**2.** Mucopolysaccharides according to Claim 1, characterized in that they have a ratio of the Yin-Wessler to USP titers of at least 6 and, advantageously, of more than 10.

**3.** Mucopolysaccharides according to Claim 1 or 2, characterized in that the terminal unit corresponds to the formula:

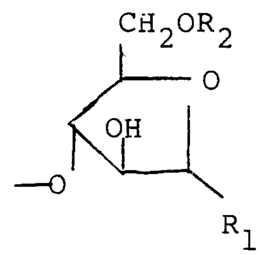

in which $R_1$ represents a functional group selected in particular among aldehyde, alcohol or carboxylic acid groups or their derivatives, particularly the acetals, amides, ethers, esters or corresponding salts, and $R_2$ represents a hydrogen atom or an $-SO_3^-$ group and the physiologically acceptable salts.

**4.** Mucopolysaccharides according to Claim 3, characterized in that $R_1$ represents a $-CH_2OH$ group.

**5.** Mucopolysaccharides according to Claim 2, characterized in that said components have a Yin-Wessler activity of about 200-270 ui/mg and a YW/USP ratio of 10-22.

**6.** Mucopolysaccharides according to Claim 2, characterized in that said components have a Yin-Wessler activity of about 250 ui/mg, a YW/USP ratio of at least 14 and units having the 2,5-anhydro-D-mannitol structure as terminal units.

**7.** Mucopolysaccharides according to Claim 2, characterized in that said components have a Yin-Wessler activity of about 270 ui/mg, a YW/USP ratio of about 22 and units having the 2,5-anhydro-D-mannonic acid structure as terminal units.

**8.** Mucopolysaccharides according to any one of Claims 1 to 7, characterized in that they are in the form of salts of calcium, of sodium or of magnesium.

**9.** Mucopolysaccharides according to one of Claims 1 to 8, characterized in that they have a molecular weight of about 3000 to 5000 daltons.

**10.** Process for obtaining mucopolysaccharides derived from heparin formed by a mixture of mucopolysaccharide chains having the following properties:

- it comprises a majority of components having modular weights of about 2000-8000 daltons,
- it is soluble in a water and alcohol medium (water and ethanol) having a titer of 55-61° GL, preferably of the order of 58° GL,
- it tends towards insolubility in a water and ethanol medium having a higher alcohol content,
- it is insoluble in pure alcohol,
- it has YW and USP titers respectively in a ratio of at least 3 with a YW activity maintained, if not increased, with respect to that of heparin,
- it comprises a majority of components having terminal units having the basic 2,5-anhydro-D-manno structure in which the primary alcohol function at 6 may or may not be substituted by an $-SO_3^-$ group, and their pharmaceutically acceptable salts,

characterized in that heparin having chains of molecular weights ranging from about 2000 to 50000 are subjected to the action of the nitrous acid formed in situ by the addition of controlled quantities of an acid to an inorganic salt of the nitrous acid, this reaction being made in an aqueous medium, at a temperature of from about 0°C to ambient, and when the degree of depolymerization is reached, depolymerization is interrupted when the mucopolysaccharides have molecular weights of 2000 to 8000, and the mucopolysaccharides having a 2,5-anhydro-manno structure at the end of chain are separated.

**11.** Process according to Claim 10, characterized in that the inorganic salt of the nitrous acid used is an alkaline or alkaline earth salt.

**12.** Process according to Claim 11, characterized in that the inorganic salt of the nitrous acid is sodium nitrite.

**13.** Process according to Claim 12, characterized in that the nitrous acid is formed in situ by the addition to the sodium nitrite of an acid having physiologically acceptable anions, such as acetic acid and, preferably, hydrochloric acid.

**14.** Process according to any one of Claims 11 to 13, characterized in that the final concentration of heparin is advantageously from 1 to 10 g, preferably from 1.5 to 5 g, and in particular approximately 2 g per 100 ml of reaction medium, the final sodium nitrite concentration being able to vary from 0.02M to 0.1M, and preferably being about 0.05M.

**15.** Process according to Claim 13 or 14, characterized in that, to form the nitrous acid in situ, hydrochloric acid is added in a controlled quantity sufficient to obtain a pH of the order of 2 to 3, advantageously from 2.2 to 2.7, preferably 2.5.

**16.** Process according to any one of Claims 10 to 15, characterized in that operation is carried out at a temperature of the order of 0 to 10°C, preferably 4°C, for a sufficient time to obtain the desired degree of depolymerization, this time being about 10 minutes when operation is carried out at 4°C.

**17.** Process according to any one of Claims 10 to 16, characterized by the supplementary step wherein the mixture of mucopolysaccharides having terminal groups with 2,5-anhydro-D-mannose structure is subjected to an operation of reduction by means of a reducing agent selected from those normally used for the transformation of aldehyde groups into alcohol groups, in particular with a metal borohydride, and the reduced products obtained whose terminal groups have the 2,5-anhydro-D-mannitol structure, are recovered.

**18.** Process according to any one of Claims 10 to 16, characterized by the supplementary step wherein the mixture of mucopolysaccharides having terminal groups with 2,5-anhydro-D-mannose structure is subjected to the action of an oxidizing agent selected from those normally used for the transformation of the aldehyde groups into acid groups and the oxidized products obtained whose terminal groups have the 2,5-anhydro-D-mannonic acid structure, are recovered.

**19.** Medicaments, characterized in that they comprise an effective quantity of at least one mucopolysaccharide formed by a mixture of mucopolysaccharide chains according to any one of Claims 1 to 9, in association with a pharmaceutical vehicle.

20. Medicaments according to Claim 19, characterized in that they are in appropriate forms for administration per os, advantageously in the form of gastro-resistant capsules, tablets or pills, or in an appropriate form for rectal administration, in the form of suppositories, or in the form of aerosols or ointments, or in the form of injectable compositions.

21. Medicaments according to Claim 20, characterized in that they are in the form of injectable pharmaceutical compositions containing from 1000 to 100000 ui (Yin-Wessler) per ml of products, preferably from 5000 to 50000, particularly 25000 ui/ml when these solutions are intended for sub-cutaneous injection and from 500 to 10000, particularly 5000 ui/ml of products when they are intended for intravenous injection or for perfusion.

**Patentansprüche**

1. Von Heparin abgeleitete Mucopolysaccharide, **dadurch gekennzeichnet**, daß sie aus einer Mischung von Mucopolysaccharidketten gebildet sind, die durch Salpetrigsäure-Depolymerisation von Heparin erhältlich ist, welche Salpetrlgsäure-Depolymerisation darin besteht, Heparin, welches Ketten mit Molekulargewichten von etwa 2000 bis etwa 50000 aufweist, der Einwirkung von durch Zugabe von gesteuerten Mengen einer Säure zu einem anorganischen Salz der Salpetrigen Säure in situ gebildeten Salpetrigen Säure auszusetzen, wobei diese Reaktion in wäßrigem Medium bei einer Temperatur von etwa 0 °C bis Raumtemperatur durchgeführt wird, die Depolymerisation dann zu unterbrechen, wenn die Mucopolysaccharide Molekulargewichte von 2000 bis 8000 aufweisen, und die in dieser Weise erhaltenen Mucopolysaccharid-Ketten abzutrennen, welche Mischung die folgenden Eigenschaften besitzt:
   - sie umfaßt eine Mehrzahl von Bestandteilen mit Molekulargewichten von etwa 2000 bis 8000 Dalton,
   - sie ist in einem wäßrig-alkoholischen Medium (Wasser-Ethanol) mit einem Titer von 55 bis 61 ° GL, vorzugsweise von 58 ° GL löslich,
   - sie neigt in einem Wasser-Ethanol-Medium mit einem höheren Alkoholgehalt zu Unlöslichkeit,
   - sie ist in reinem Alkohol unlöslich,
   - sie besitzt YW- beziehungsweise USP-Titer in einem Verhältnis von mindestens 3 bei einer YW-Aktivltät, die im Vergleich zu der des Heparins beibehalten, wenn nicht erhöht ist,
   - sie umfaßt eine Mehrzahl von Bestandteilen mit Endgruppen, welche die 2,5-Anhydro-D-manno-Grundstruktur aufweisen, worin die primäre Alkoholfunktion in der 6-Stellung gegebenenfalls durch eine $-SO_3^-$-Gruppe substituiert ist, sowie deren pharmazeutisch annehmbare Salze.

2. Mucopolysaccharide nach Anspruch 1, **dadurch gekennzeichnet**, daß sie ein Verhältnis des Yin-Wessler-Titers zu dem USP-Titer von mindestens 6, vortellhafterweise von mehr als 10 besitzen.

3. Mucopolysaccharide nach Anspruch 1 oder 2, **dadurch gekennzeichnet**, daß die Endgruppe der Formel:

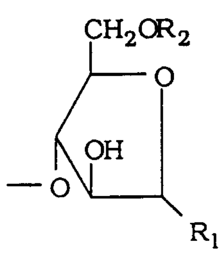

entspricht, worin $R_1$ eine funktionelle Gruppe darstellt ausgewählt insbesondere aus Aldehydgruppen. Alkoholgruppen oder Carbonsäuregruppen oder Derivaten davon, insbebesondere Acetalen, Amiden, Ethern, Estern oder entsprechenden Salzen, und $R_2$ ein Wasserstoffatom oder eine $-SO_3^-$-Gruppe bedeuten, sowie deren physiologisch annehmbare Salze.

4. Mucopolysaccharide nach Anspruch 3, **dadurch gekennzeichnet**, daß $R_1$ eine $-CH_2OH$-Gruppe darstellt.

5. Mucopolysaccharide nach Anspruch 2, **dadurch gekennzeichnet**, daß die genannten Bestandteile eine Yin-Wessler-Aktivität von etwa 200 bis 270 iE/mg und ein YW/USP-Verhältnis von 10 bis 22 aufweisen.

6. Mucopolysaccharide nach Anspruch 2, **dadurch gekennzeichnet**, daß die genannten Bestandteile eine Yin-Wessler-Aktivität von etwa 250 iE/mg, ein YW/USP-Verhältnis von mindestens 14 und Reste der 2,5-Anhydro-D-mannit-Struktur als Endgruppen aufweisen.

7. Mucopolysaccharide nach Anspruch 2, **dadurch gekennzeichnet**, daß die genannten Bestandteile eine YIN-Wessler-Aktivität von etwa 270 iE/mg, ein YW/USP-Verhältnis von etwa 22 und Reste der 2,5-Anhydro-D-mannonsäure-Struktur als Endgruppen aufweisen.

8. Mucopolysaccharide nach einem der Ansprüche 1 bis 7, **dadurch gekennzeichnet**, daß sie in Form von Calcium-, Natrium- oder Magnesium-Salzen vorliegen.

9. Mucopolysaccharide nach einem der Ansprüche 1 bis 8, **dadurch gekennzeichnet**, daß sie ein Molekulargewicht von etwa 3000 bis 5000 Dalton aufweisen.

10. Verfahren zur Herstellung von Mucopolysacchariden, die aus Heparin abgeleitet sind und aus einer Mischung von Mucopolysaccharid-Ketten gebildet sind, welche die folgenden Eigenschaften besitzt:
    - sie umfaßt eine Mehrzahl von Bestandteilen mit Molekulargewichten von etwa 2000 bis 8000 Dalton,
    - sie ist in einem wäßrig-alkoholischen Medium (Wasser-Ethanol) mit einem Titer von 55 bis 61° GL, vorzugsweise von 58° GL löslich.
    - sie neigt in einem Wasser-Ethanol-Medium mit einem höheren Alkoholgehalt zu Unlöslichkeit,
    - sie ist in reinem Alkohol unlöslich,
    - sie besitzt YW- beziehungsweise USP-Titer in einem Verhältnis von mindestens 3 bei einer YW-Aktivität, die im Vergleich zu der des Heparins beibehalten, wenn nicht erhöht ist,
    - sie umfaßt eine Mehrzahl von Bestandteilen mit Endgruppen, welche die 2,5-Anhydro-D-manno-Grundstruktur aufweisen, worin die primäre Alkoholfunktion in der 6-Stellung gegebenenfalls durch eine $-SO_3^-$-Gruppe substituiert ist, und von deren pharmazeutisch annehmbaren Salzen,

    **dadurch gekennzeichnet**, daß man Heparin mit Ketten mit Molekulargewichten von etwa 2000 bis 50 000 der Einwirkung von durch Zugabe von gesteuerten Mengen einer Säure zu einem anorganischen Salz der Salpetrigen Säure in situ gebildeter Salpetriger Säure unterwirft, wobei diese Reaktion in wäßrigem Medium bei einer Temperatur von 0°C bis Raumtemperatur durchgeführt wird, und, wenn der Depolymerisationsgrad erreicht ist, man die Depolymerisation unterbricht, wenn die Mucopolysaccharide Molekulargewichte von 2000 bis 8000 aufweisen, und man die am Ende der Kette eine 2,5-Anhydro-manno-Struktur aufweisenden Mucopolysaccharide abtrennt.

11. Verfahren nach Anspruch 10, **dadurch gekennzeichnet**, daß das eingesetzte anorganische Salz der Salpetrigen Säure ein Alkali- oder Erdalkalisalz ist.

12. Verfahren nach Anspruch 11, **dadurch gekennzeichnet**, daß das anorganische Salz der Salpetrigen Säure Natriumnitrit ist.

13. Verfahren nach Anspruch 12, **dadurch gekennzeichnet**, daß die Salpetrige Säure in situ durch Zugabe einer Säure mit physiologisch annehmbaren Anionen, wie Essigsäure und vorzugsweise Chlorwasserstoffsäure, zu Natriumnitrit gebildet wird.

14. Verfahren nach einem der Ansprüche 11 bis 13, **dadurch gekennzeichnet**, daß die Endkonzentration an Heparin mit Vorteil 1 bis 10g. bevorzugter 1,5 bis 5g und insbesondere 2g pro 100 ml des Reaktionsmediums beträgt, wobei die Endkonzentration an Natriumnitrit zwischen 0,02M bis 0,1 M variieren kann und vorzugsweise etwa 0,05 M beträgt.

15. Verfahren nach Anspruch 13 oder 14, **dadurch gekennzeichnet**, daß man zur in situ-Bildung der Salpetrigen Säure Chlorwasserstoffsäure in gesteuerter und ausreichender Menge zum Erhalt eines pH-Wertes im Bereich von 2 bis 3, mit Vorteil im Bereich von 2,2 bis 2,7 und vorzugsweise von 2,5 zugibt.

**16.** Verfahren nach einem der Ansprüche 10 bis 15, **dadurch gekennzeichnet**, daß man bei einer Temperatur im Bereich von 0 bis 10 ° C, vorzugsweise von 4 ° C, während einer Zeitdauer arbeitet. die zum Erreichen des gewünschten Depolymerisationsgrades ausreicht, wobei diese Zeitdauer etwa 10 Minuten beträgt. wenn man bei 4 ° C arbeitet.

**17.** Verfahren nach einem der Ansprüche 10 bis 16, **gekennzeichnet durch** die zusätzliche Stufe, bei der man die Mischung der Mucopolysaccharide mit Endgruppen der 2,5-Anhydro-D-mannose-Struktur einer Reduktionsbehandlung mit Hilfe eines Reduktionsmittels, ausgewählt aus den üblicherweise bei der Umwandlung von Aldehydgruppen zu Alkoholgruppen verwendeten Reduktionsmitteln, insbesondere eines Metallborhydrids. unterwirft und die erhaltenen Reduktionsprodukte. deren Endgruppen die 2,5-Anhydro-D-mannit-Struktur aufweisen, gewinnt.

**18.** Verfahren nach einem der Ansprüche 10 bis 16, **gekennzeichnet durch** die zusätzliche Stufe, bei der man die Mischung der Mucopolysaccharide mit Endgruppen der 2,5-Anhydro-D-mannose-Struktur der Einwirkung eines Oxidationsmittels, ausgewählt aus den üblicherweise für die Umwandlung von Aldehydgruppen in Säuregruppen verwendeten Oxidationsmitteln, unterwirft und die erhaltenen Oxidationsprodukte, deren Endgruppen die 2,5-Anhydro-D-mannonsäure-Struktur aufweisen, gewinnt.

**19.** Arzneimittel, **dadurch gekennzeichnet**, daß sie eine wirksame Menge mindestens eines aus einer Mischung aus Mucopolysaccharidketten gebildeten Mucopolysaccharids nach einem der Ansprüche 1 bis 9, zusammen mit einem pharmazeutischen Trägermaterial, enthalten.

**20.** Arzneimittel nach Anspruch 19, **dadurch gekennzeichnet**, daß sie in einer für die Verabreichung auf oralem Wege geeigneten Form, mit Vorteil in Form von magensäurebeständigen Gelkapseln, Kompretten oder Tabletten, Pillen, oder auch in einer für die rektale Verabreichung geeigneten Form, als Suppositorien, oder auch in Form von Aerosolen oder Salben oder auch in Form injizierbarer Zusammensetzungen vorliegen.

**21.** Arzneimittel nach Anspruch 20, **dadurch gekennzeichnet**, daß sie in Form von injizierbaren pharmazeutischen Zusammensetzungen vorliegen, die 1000 bis 100 000 iE (Yin-Wessler) pro ml der Produkte, vorzugsweise 5000 bis 50 000 und insbesondere 25 000 iE/ml enthalten, wenn diese Lösungen für die subkutane Injektion bestimmt sind, und 500 bis 10 000, insbesondere 5000 iE/ml der Produkte, wenn sie für die intravenöse Injektion oder für die Perfusion bestimmt sind.